# EUROPÄISCHE PATENTSCHRIFT

(11) **EP 2 404 689 B1**
(45) Veröffentlichungstag und Bekanntmachung des Hinweises auf die Patenterteilung: **22.03.2017**
(21) Anmeldenummer: 11004276.9
(22) Anmeldetag: 24.05.2011
(51) Int. Cl.: B23C 5/10, A61B 17/16, A61C 3/02

(54) **Dentalwekzeug**
Dental tool
Outil dentaire

(30) Priorität: 07.07.2010 DE 102010026334
(43) Veröffentlichungstag der Anmeldung: 11.01.2012
(73) Patentinhaber: Gebr. Brasseler GmbH & Co. KG, D-32657 Lemgo (DE)
(72) Erfinder: Meier, Friedrich Wilhelm, 32825 Blomberg (DE); Niemeier, Markus, 32791 Lage (DE)
(74) Vertreter: Hoefer & Partner Patentanwälte mbB

(56) Entgegenhaltungen:
- DE-C- 555 440
- DE-U1-202007 018 284
- GB-A- 753 233
- GB-A- 2 405 365
- JP-A- 63 047 007

## Beschreibung

Die Erfindung bezieht sich auf ein Dentalwerkzeug gemäß den Merkmalen des Oberbegriffs des Anspruches 1.

Im Einzelnen bezieht sich die Erfindung auf ein chirurgisches Werkzeug bzw. einen Dentalfräser mit einem an einem drehbaren Schaft gelagerten Arbeitskopf, wobei der Arbeitskopf mit Schneiden unterschiedlicher Verzahnungen versehen ist.

Aus dem Stand der Technik sind Dentalfräser oder chirurgische Fräser bekannt, bei welchen eine Grundverzahnung vorgesehen ist, die über den gesamten Umfang des Arbeitskopfes gleichmäßig angeordnet ist. Zusätzlich zu dieser Grundverzahnung kann eine Kreuzverzahnung aufgebracht werden, welche in gleicher oder in entgegengesetzter Drallrichtung zu der Grundverzahnung ausgerichtet ist. Auch diese Kreuzverzahnung ist über den gesamten Umfang gleichmäßig verteilt. Ergänzend kennt der Stand der Technik auch Ausgestaltungen, bei welchen zusätzlich eine Spanbrechernut schraubenlinienartig längs des Arbeitskopfes vorgesehen ist, um die Spanlängen und Spanbreiten zu verringern.

Die 20 2007 018 284 U1 beschreibt einen Dentalfräser mit einem Fräskopf, welcher mit Schneiden versehen ist, wobei an einzelnen kreissektorartigen Teilbereichen, welche jeweils einen Winkelbereich von ungefähr 90° aufweisen, bezogen auf eine achsensenkrechte Schnittebene, welche sich nur über einen Teil des Umfangs des Arbeitskopfes erstrecken, in Umfangsrichtung alternierend Schneiden einer ersten Verzahnung und Schneiden einer zweiten Verzahnung ausgebildet sind, und dass stirnseitig an dem Arbeitskopf kreissektorartige Bereiche ausgebildet sind, welche jeweils in Umfangsrichtung alternierend aus den einlaufenden Schneiden der ersten Verzahnung oder den einlaufenden Schneiden der zweiten Verzahnung gebildet sind. Die Schneiden sind zusätzlich durch Spanbrechernuten unterbrochen.

Die GB 2 405 365 A zeigt ein Dentalwerkzeug, bei welchem um den Umfang alternierend unterschiedliche Verzahnungen vorgesehen sind. Es sind dabei insbesondere zwei Verzahnungsgruppen vorgesehen, welche jeweils drei gedrallte Schneiden haben.

Der Erfindung liegt die Aufgabe zugrunde, ein Dentalwerkzeug bzw. ein chirurgisches Werkzeug zu schaffen, welches bei einfachem Aufbau und einfacher, kostengünstiger Herstellbarkeit eine gute Abtragsleistung sowie eine hohe Laufruhe aufweist.

Erfindungsgemäß wird die Aufgabe durch die Merkmalskombination des Anspruchs 1 gelöst, die Unteransprüche zeigen weitere vorteilhafte Ausgestaltungen der Erfindung.

Erfindungsgemäß ist somit vorgesehen, dass zunächst am gesamten Umfang des Arbeitskopfes Schneiden einer ersten Verzahnung ausgebildet sind. Diese Schneiden verlaufen bevorzugt zueinander parallel und sind am Umfang gleichmäßig verteilt. Im stirnseitigen Bereich verlaufen diese in der Art einer Gruppenverzahnung weiterhin im Wesentlichen parallel zueinander.

Erfindungsgemäß ist nun vorgesehen, dass an sektorartigen Teilbereichen des Arbeitskopfes, d.h., an Teilbereichen, welche sich nur über einen Teil des Umfangs erstrecken, zusätzlich zu der ersten Verzahnung Schneiden einer zweiten Verzahnung ausgebildet sind.

In besonders günstiger Ausgestaltung der Erfindung ist entweder vorgesehen, die erste und die zweite Verzahnung mit gleicher Drallrichtung oder mit entgegengesetzter Drallrichtung auszubilden.

Über den Umfang des Arbeitskopfes ist somit erfindungsgemäß in einzelnen Kreissektoren (bezogen auf eine achsensenkrechte Schnittebene) vorgesehen, dass eine erste und eine zweite Verzahnung vorhanden sind und dass in einzelnen Sektoren nur die erste Verzahnung ausgebildet ist.

Das erfindungsgemäße Dentalwerkzeug zeichnet sich durch eine verbesserte Laufruhe aus und verfügt über eine verbesserte Abtragsleistung, wobei die bearbeitete Oberfläche eine höhere Oberflächenqualität aufweist.

Stirnseitig weist das erfindungsgemäße Dentalwerkzeug ebenfalls kreissektorartige Bereiche auf, in welchen (in Umfangsrichtung alternierend) die Schneiden der ersten Verzahnung bzw. die Schneiden der zweiten Verzahnung enden. Somit weist auch das stirnseitige Ende des erfindungsgemäßen Dentalwerkzeugs ein verbessertes Schnittverhalten auf.

In besonders günstiger Ausgestaltung der Erfindung ist vorgesehen, dass der Arbeitskopf eine zylindrische Grundform aufweist und/oder stirnseitig abgerundet ist.

In stirnseitiger Ansicht führt die kreissektorartige Anordnung der zweiten Verzahnung dazu, dass stirnseitig Kreissektoren oder Quadranten ausgebildet sind, welche jeweils aus den einlaufenden Schneiden der ersten Verzahnung bzw. den einlaufenden Schneiden der zweiten Verzahnung gebildet sind.

Im Folgenden wird die Erfindung anhand von Ausführungsbeispielen in Verbindung mit der Zeichnung beschrieben. Dabei zeigt:
- Fig. 1: eine Seitenansicht eines ersten Ausführungsbeispiels der Erfindung,
- Fig. 2: eine perspektivische Ansicht des Endbereichs des in Fig. 1 gezeigten Ausführungsbeispiels,
- Fig. 3: eine perspektivische Darstellung, analog Fig. 2, mit entgegengesetzter Drallrichtung der ersten und der zweiten Verzahnung,
- Fig. 4: eine stirnseitige Ansicht des Ausführungsbeispiels der Fig. 1 und 2,
- Fig. 5: eine weitere stirnseitige Ansicht, analog Fig. 4,
- Fig. 6: eine Schnittansicht des Ausführungsbeispiels der Fig. 3, und
- Fig. 7: eine Schnittansicht des Ausführungsbeispiels der Fig. 1, 2, 4 bzw. 5.

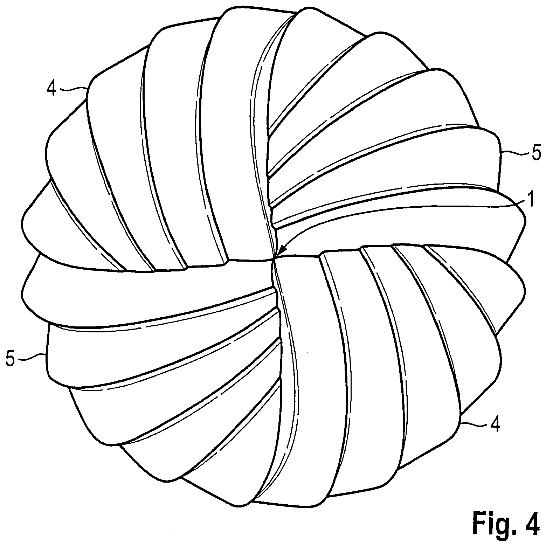

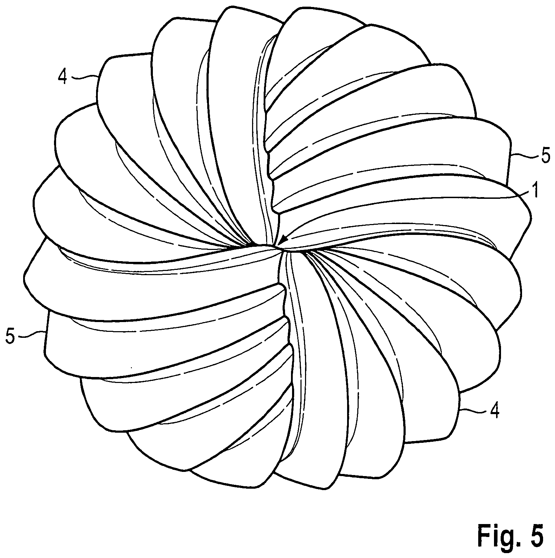

Das erfindungsgemäße Dentalwerkzeug weist eine zylindrische Grundform auf, welche am stirnseitigen Ende im Wesentlichen halbkugelförmig abgerundet ist. Diese Grundform wird zunächst aus einem geeigneten Material mechanisch erzeugt. In einem ersten Arbeitsschritt werden dann mittels zumindest einer Schleifscheibe zur Erzeugung eines Arbeitskopfes 3 erste Schneiden 4 einer ersten Verzahnung gefräst. Bei den gezeigten Ausführungsbeispielen ist die erste Verzahnung 4 jeweils gedrallt. Bei Blick auf das stirnseitige Ende (siehe Fig. 4 und 5) erfolgt eine Drehung um eine Drehachse 1 in Uhrzeigergegenrichtung. Entsprechend werden die Schneidengeometrien ausgewählt.

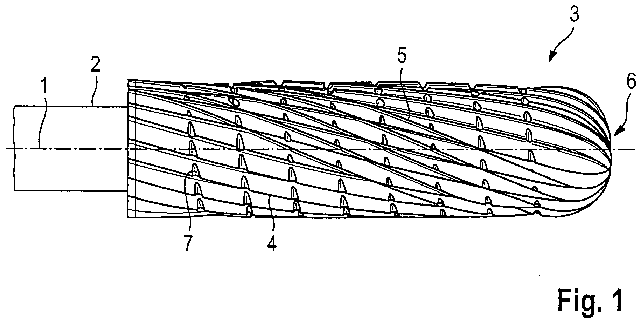

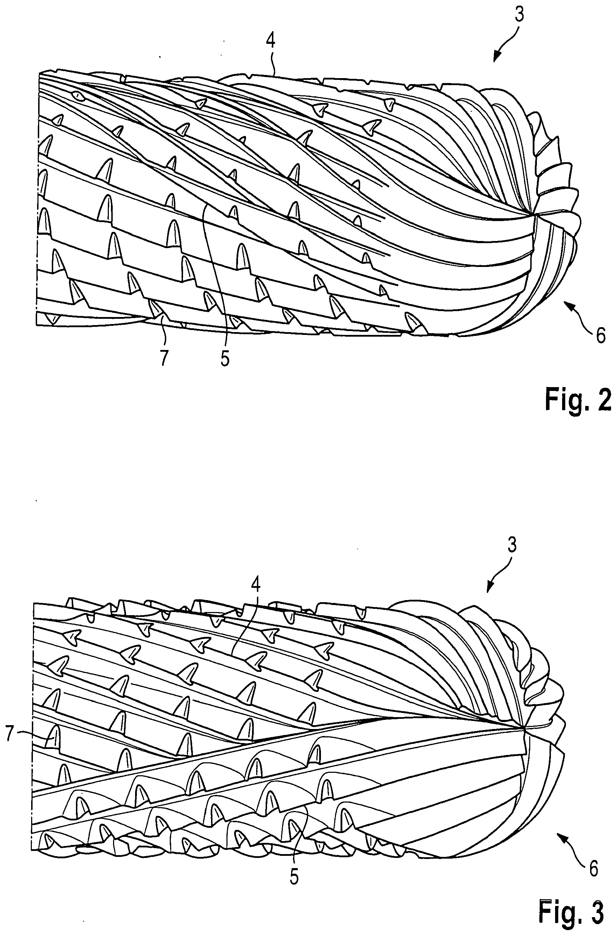

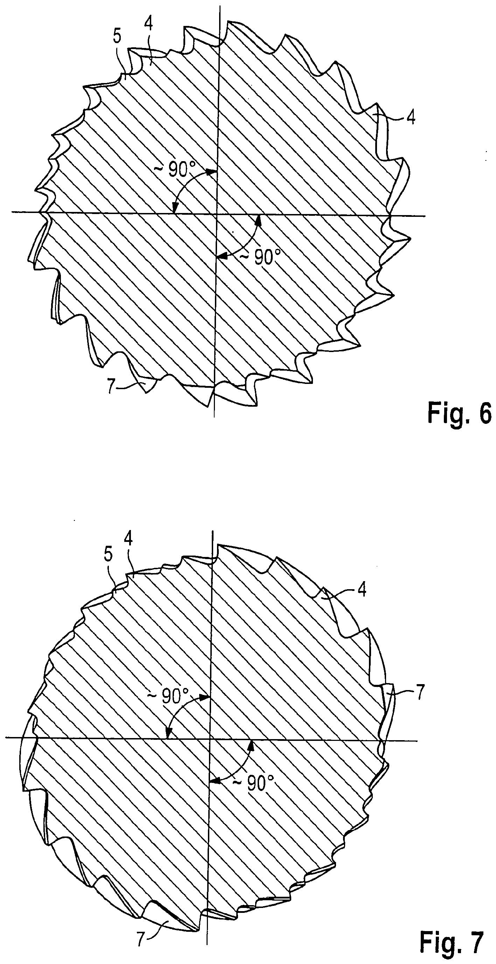

Nach Aufbringung der ersten Verzahnung 4 durch gleichmäßige Erzeugung der Schneiden 4 in zueinander paralleler Ausrichtung und mit gleichem Abstand um den gesamten Umfang des Arbeitskopfes 3 erfolgt in zwei Kreissektoren, die jeweils einen Winkelbereich von ungefähr 90° aufweisen (siehe Fig. 7), die Aufbringung einer zweiten Verzahnung 5 mit Schneiden 5, welche ebenfalls einen Rechtsdrall haben. Hierdurch wird das in den Fig. 1, 2, 4 und 7 gezeigte Ausführungsbeispiel erzeugt. Auch hierbei wird, beginnend vom stirnseitigen Endbereich, mittels eines Fräswerkzeugs die zweite Verzahnung 5 gefräst. Diese überlagert somit Teilbereiche der ersten Verzahnung, so wie dies insbesondere aus den Fig. 1 und 2 ersichtlich ist.

Nachfolgend erfolgt die Einbringung einer Spanbrechernut 7, welche spiralförmig um den zylindrischen Bereich des Arbeitskopfes 3 angeordnet ist.

Es ergibt sich somit, dass über den Umfang des Arbeitskopfes 3 bei Drehung um die Drehachse 1 alternierend jeweils über einen Umfangsbereich von ca. 90° nur die erste Verzahnung 4 bzw. eine Kombination aus der ersten Verzahnung 4 und der zweiten Verzahnung 5 in Eingriff gelangen.

Die Fig. 3 und 6 zeigen ein weiteres Ausführungsbeispiel, bei welchem die zweite Verzahnung 5 mit einem Linksdrall aufgebracht ist, so wie dies insbesondere aus Fig. 3 ersichtlich ist. Weiterhin zeigt die Fig. 3, dass sich auch bei diesem Ausführungsbeispiel bei Blick auf eine Stirnseite 6 vier Quadranten ergeben, die jeweils entweder nur die erste Verzahnung 4 oder eine Kombination aus der ersten Verzahnung 4 und der zweiten Verzahnung 5 aufweisen, so wie dies die Fig. 6 in einer, analog zur Fig. 7, senkrecht zur Drehachse 1 ausgeführten Schnittebene zeigt.

Die Fig. 4 und 5 zeigen, dass die Schneiden der ersten Verzahnung stirnseitig in Sektoren (4 im Beispiel gemäß Fig. 4 und 5) unterteilt sind und die jeweils gegenüberliegenden Sektoren identisch auslaufend angeordnete Schneiden aufweisen, wobei zwischen den Sektoren ein Wechsel zwischen parallel und mittig (Sektoren links oben und rechts unten in Fig. 4 und 5) auslaufend angeordneten Schneiden stattfindet.

Die Fig. 5 zeigt eine Ansicht, analog Fig. 4. Dieses Ausführungsbeispiel der Fig. 5 unterscheidet sich von dem Ausführungsbeispiel der Fig. 4 darin, dass die Schneiden abwechselnd parallel bzw. zur Mitte auslaufend angeordnet sind. Fig. 5 zeigt somit in dem Quadranten rechts oben und links unten parallel auslaufende Schneiden, während in den beiden anderen Quadranten (rechts unten und links oben gemäß Fig. 5) die Schneiden mittig auslaufen.

Weiterhin ist es erfindungsgemäß möglich, die erste Verzahnung und die zweite Verzahnung unterschiedlich auszubilden, beispielsweise mit unterschiedlichen Schneidwinkeln, Keilwinkeln oder Freiwinkeln sowie mit unterschiedlicher Tiefe der Spannut.

### Bezugszeichenliste

- 1: Drehachse
- 2: Schaft
- 3: Arbeitskopf
- 4: Schneide/erste Verzahnung
- 5: Schneide/zweite Verzahnung
- 6: Stirnseite
- 7: Spanbrechernut

## Patentansprüche

1. Dentalwerkzeug mit einem um eine Drehachse (1) rotierbaren, an einem Schaft (2) befestigten Arbeitskopf (3), welcher mit Schneiden (4, 5) versehen ist,
wobei am gesamten Umfang des Arbeitskopfes (3) gleichmäßig verteilt Schneiden (4) einer ersten Verzahnung ausgebildet sind,
wobei an einzelnen kreissektorartigen Teilbereichen, welche jeweils einen Winkelbereich von ungefähr 90° aufweisen, bezogen auf eine achsensenkrechte Schnittebene, welche sich nur über einen Teil des Umfangs des Arbeitskopfes (3) erstrecken, zusätzlich zu der ersten Verzahnung (4) Schneiden (5) einer zweiten Verzahnung ausgebildet sind, und wobei stirnseitig (6) an dem Arbeitskopf (3) kreissektorartige Bereiche ausgebildet sind, welche jeweils in Umfangsrichtung alternierend aus den einlaufenden Schneiden (4) der ersten Verzahnung oder den einlaufenden Schneiden (5) der zweiten Verzahnung gebildet sind.

2. Dentalwerkzeug nach Anspruch 1, **dadurch gekennzeichnet, dass** die erste und/oder die zweite Verzahnung (4, 5) gedrallt ausgebildet sind.

3. Dentalwerkzeug nach Anspruch 2, **dadurch gekennzeichnet, dass** eine gleiche Drallrichtung vorliegt.

4. Dentalwerkzeug nach Anspruch 2, **dadurch gekennzeichnet, dass** eine entgegengesetzte Drallrichtung vorliegt.

5. Dentalwerkzeug nach einem der Ansprüche 1 bis 4, **dadurch gekennzeichnet, dass** die Schneiden (4, 5) der jeweiligen Verzahnung jeweils parallel zueinander angeordnet sind.

6. Dentalwerkzeug nach einem der Ansprüche 1 bis 5, **dadurch gekennzeichnet, dass** die Schneiden (5) der zweiten Verzahnung stirnseitig (6) parallel auslaufend angeordnet sind, und/oder dass die Schneiden (4) der ersten Verzahnung stirnseitig (6) parallel auslaufend angeordnet sind.

7. Dentalwerkzeug nach einem der Ansprüche 1 bis 5, **dadurch gekennzeichnet, dass** die Schneiden (4) der ersten Verzahnung und die Schneiden (5) der zweiten Verzahnung jeweils zur Mitte auslaufen oder dass nur die Schneiden (4) der ersten Verzahnung zur Mitte auslaufen, wobei in Umfangsrichtung alternierend jeweils ein Sektor mit mittig auslaufenden Schneiden und ein benachbarter Sektor mit parallel auslaufenden Schneiden ausgebildet ist.

8. Dentalwerkzeug nach einem der Ansprüche 1 bis7, **dadurch gekennzeichnet, dass** an dem Arbeitskopf (3) zumindest eine Spanbrechernut (7) ausgebildet ist.

9. Dentalwerkzeug nach einem der Ansprüche 1 bis 8, **dadurch gekennzeichnet, dass** der Arbeitskopf (3) eine zylindrische Grundform aufweist und/oder dass der Arbeitskopf stirnseitig (6) eine abgerundete Grundform aufweist.

## Claims

1. Dental tool comprising a work head (3) which is rotatable about an axis of rotation (1), is fixed to a shaft (2), and is provided with blades (4, 5),
wherein uniformly distributed blades (4) of a first toothing are formed on the entire circumference of the work head (3),
wherein blades (5) of a second toothing are formed in addition to the first toothing (4) on individual circle-sector-like sub-regions, which each have an angular range of approximately 90° with respect to a section plane perpendicular to the axis and which extend only over part of the circumference of the work head (3), and
wherein circle-sector-like regions are formed the on the end face (6) of the work head (3), and are each formed, alternately in the circumferential direction, from the incoming blades (4) of the first toothing or the converging blades (5) of the second toothing.

2. Dental tool according to claim 1, **characterised in that** the first and/or the second toothing (4, 5) are formed twisted.

3. Dental tool according to claim 2, **characterised in that** an identical direction of twist is provided.

4. Dental tool according to claim 2, **characterised in that** an opposite direction of twist is provided.

5. Dental tool according to any of claims 1 to 4, **characterised in that** the blades (4, 5) of each toothing are arranged mutually parallel in each case.

6. Dental tool according to any of claims 1 to 5, **characterised in that** the blades (5) of the second toothing are arranged extending out parallel at the end face (6) and/or **in that** the blades (4) of the first toothing are arranged extending out parallel at the end face (6).

7. Dental tool according to any of claims 1 to 5, **characterised in that** the blades (4) of the first toothing and the blades (5) of the second toothing each extend out with respect to the centre or **in that** only the blades (4) of the first toothing extend out with respect to the centre, in each case alternately one sector being formed with blades extending out centrally and an adjacent sector being formed with blades extending out parallel.

8. Dental tool according to any of claims 1 to 7, **characterised in that** at least one chip breaker groove (7) is formed on the work head (3).

9. Dental tool according to any of claims 1 to 8, **characterised in that** the work head (3) has a cylindrical base shape and/or **in that** the work head has a rounded base shape at the end face (6).

## Revendications

1. Outil dentaire avec une tête de travail (3) pouvant tourner autour d'un axe de rotation (1), fixée au niveau d'une tige (2), laquelle est pourvue de lames (4, 5),
dans lequel des lames (4) d'une première denture sont réalisées avec une répartition homogène au niveau de l'ensemble de la périphérie de la tête de travail (3),
dans lequel en plus de la première denture (4), des lames (5) d'une deuxième denture sont réalisées au niveau de certaines zones partielles de type secteur circulaire, qui présentent chacune une plage angulaire d'environ 90°, par rapport à un plan de découpe perpendiculaire axial, lesquelles s'étendent seulement sur une partie de la périphérie de la tête de travail (3), et dans lequel sont réalisées, côté frontal (6), au niveau de la tête de travail (3), des zones de type secteur circulaire, qui sont formées respectivement en alternance dans la direction périphérique à partir des lames (4) rentrant de la première denture ou des lames (5) rentrant de la deuxième denture.

2. Outil dentaire selon la revendication 1, **caractérisé en ce que** la première et/ou la deuxième denture (4, 5) sont réalisées de manière vrillée.

3. Outil dentaire selon la revendication 2, **caractérisé en ce qu'**une direction de vrillage identique est présente.

4. Outil dentaire selon la revendication 2, **caractérisé en ce qu'**une direction de vrillage opposée est présente.

5. Outil dentaire selon l'une quelconque des revendications 1 à 4, **caractérisé en ce que** les lames (4, 5) de la denture respective sont disposées respectivement de manière parallèle les unes par rapport aux autres.

6. Outil dentaire selon l'une quelconque des revendications 1 à 5, **caractérisé en ce que** les lames (5) de la deuxième denture sont disposées côté frontal (6) de manière à converger de manière parallèle, et/ou **en ce que** les lames (4) de la première denture sont disposées côté frontal (6) de manière à converger de manière parallèle.

7. Outil dentaire selon l'une quelconque des revendications 1 à 5, **caractérisé en ce que** les lames (4) de la première denture et les lames (5) de la deuxième denture convergent respectivement en direction du centre, ou **en ce que** seules les lames (4) de la première denture convergent en direction du centre, dans lequel respectivement un secteur avec des lames convergeant au centre et un secteur adjacent avec des lames convergeant de manière parallèle sont réalisés en alternance dans la direction périphérique.

8. Outil dentaire selon l'une quelconque des revendications 1 à 7, **caractérisé en ce qu'**au moins une rainure brise-copeaux (7) est réalisée au niveau de la tête de travail (3).

9. Outil dentaire selon l'une quelconque des revendications 1 à 8, **caractérisé en ce que** la tête de travail (3) présente une forme de base cylindrique, et/ou **en ce que** la tête de travail présente, côté frontal (6), une forme de base arrondie.
